# FASCICULE DE BREVET EUROPEEN

(11) **EP 2 299 948 B1**
(45) Date de publication et mention de la délivrance du brevet: **05.08.2015**
(21) Numéro de dépôt: 09772712.7
(22) Date de dépôt: 09.06.2009
(51) Int. Cl.: A61F 5/02, A61F 5/03

(54) **DISPOSITIF DE SOUTIEN, NOTAMMENT LOMBAIRE**
STÜTZVORRICHTUNG, INSBESONDERE ZUR UNTERSTÜTZUNG DER LENDENWIRBELSÄULE
SUPPORT DEVICE, PARTICULARLY FOR LUMBAR SUPPORT

(30) Priorité: 09.06.2008 FR 0853805
(43) Date de publication de la demande: 30.03.2011
(73) Titulaire: THUASNE, 92300 Levallois Perret (FR)
(72) Inventeur: PETIOT, Séverine, 42230 Roche La Moliere (FR); DE MONCUIT, Henri, 42000 Saint Etienne (FR)
(74) Mandataire: Balesta, Pierre
(86) Numéro de dépôt international: PCT/FR2009/051085
(87) Numéro de publication internationale: WO 2010/001030

(56) Documents cités:
- WO-A-2007/129950
- DE-A1- 2 334 500
- FR-A- 2 687 912
- GB-A- 136 409
- US-A- 1 893 960
- US-A- 3 717 143

## Description

La présente invention est dans le domaine technique des dispositifs de soutien, notamment lombaire, comportant une ceinture élastique, munie à ses extrémités de moyens complémentaires de fermeture.

Une ceinture de soutien, notamment lombaire, est adaptée à la morphologie du patient et à la pathologie à traiter, en particulier pour soutenir le bas de la colonne vertébrale et les reins. Afin d'assurer sa fonction de soutien, la ceinture comporte des éléments de renfort, généralement des baleines dans un métal malléable ou dans des résines synthétiques, souvent cousus sur la ceinture dans une position légèrement inclinées en sorte de suivre la ligne virtuelle reliant les hanches à la taille. Ces éléments de renfort sont fixés sur la ceinture selon un angle d'inclinaison déterminé par le fabricant quelque soit la morphologie du patient, par exemple en « V ». Il n'est pas possible avec les ceintures de l'état de la technique d'obtenir un soutien personnalisé en permettant que les éléments de renfort adoptent une inclinaison fonction de la morphologie du patient lorsque ce dernier ajuste puis ferme la ceinture sur sa taille. Ces éléments de renfort ne s'adaptant pas à la morphologie unique de chaque patient (fonction de la taille, du poids, du sexe,...) peuvent s'avérer inconfortables, en particulier en cas de port prolongé ; à tel point que le patient ne respecte pas le temps de pose et retire sa ceinture voir ne la serre pas assez. L'effet thérapeutique escompté n'est alors pas atteint.

On connaît par le document US 1.893.960 un dispositif de soutien abdominal, comportant une ceinture élastique, munie à ses extrémités de moyens complémentaires de fermeture, et plusieurs éléments de renfort longilignes disposés selon la hauteur de la ceinture et dont les extrémités inférieures sont fixées à une plaque rigide. La mise en extension longitudinale de la ceinture provoque l'écartement des parties supérieures des éléments de renfort longilignes les unes par rapport aux autres.

WO 2007/129950 divulgue également une orthèse de dos selon le préambule de la revendication 1.

Le but de la présente invention est de proposer un dispositif de soutien, notamment lombaire qui, par rapport au dispositif connu par le document WO 2007/129950, améliore le confort du patient.

Ce but est parfaitement atteint par le dispositif de la présente invention, défini dans la revendication 1. Il s'agit d'un dispositif de soutien, notamment lombaire, comportant une ceinture élastique, munie à ses extrémités de moyens complémentaires de fermeture, et au moins deux éléments de renfort latéraux et longilignes fixés sur la hauteur de la ceinture, entre lesquels l'élasticité de la ceinture est différenciée selon sa hauteur. De manière caractéristique, ce dispositif comporte un plastron dorsal qui s'étend sur la hauteur de la ceinture et dont la partie supérieure est reliée uniquement aux extrémités supérieures desdits éléments de renfort latéraux en sorte de faire office de pièce de blocage apte à limiter l'extensibilité de la ceinture en sorte que la mise en extension longitudinale de la ceinture provoque le déplacement angulaire des extrémités inférieures desdits éléments de renfort longilignes vers l'une des desdites extrémités de la ceinture.

Le confort du patient est amélioré, d'une part, grâce à la présence du plastron dorsal et à l'absence de plaque rigide et, d'autre part, grâce au fait que les éléments de renfort latéraux adoptent une inclinaison adaptée à la morphologie du patient correspondant aux lignes virtuelles reliant les hanches à la taille.

Les moyens complémentaires de fermeture peuvent être n'importe quels moyens de fermeture connus dans l'état de la technique, et sont de préférence des organes mâle et femelle du type à boucles et à crochets, les boucles étant de préférence formées par un velours gratté, type astrakan.

La hauteur de la ceinture est définie comme étant la distance, parfois non constante, séparant le bord supérieur du bord inférieur de la ceinture.

Par mise en extension longitudinale, on comprend que le patient lorsqu'il ajuste la ceinture à sa taille, celui-ci étant débout, exerce une traction sur chacune des extrémités pour amener ces dernières à coopérer ensemble pour leur fermeture et ajuster par la même occasion le serrage de la ceinture.

De préférence, les éléments de renfort longilignes ne sont pas élastiques.

Les éléments de renfort longilignes peuvent être dans une position légèrement inclinée par rapport à la verticale du patient lorsque celui-ci est debout et porte la ceinture selon la présente invention. De préférence, les éléments de renfort longilignes sont reliés au plastron dorsal en sorte d'être sensiblement orientés à la verticale du patient lorsque ce dernier est debout.

La différenciation de l'élasticité selon la hauteur de la ceinture est obtenue par la présence du plastron dorsal dont la partie supérieure qui relie les extrémités des éléments de renfort latéraux fait office de pièce de blocage sur une partie de la hauteur de la ceinture. Ladite pièce de blocage présente une élasticité au moins dans le sens longitudinal plus faible que l'élasticité longitudinale de la ceinture en sorte que la mise en extension longitudinale de la ceinture provoque le déplacement angulaire des éléments de renfort.

Lorsque l'usager ajuste la ceinture à sa taille, il exerce une traction sur chacune de ses extrémités, générant des forces de traction qui s'exercent de part et d'autre des éléments de renfort de façon différenciée, dans une région de la longueur de la ceinture, grâce à l'élasticité différenciée sur la hauteur de la ceinture dans ladite région. En effet, la première zone la moins élastique de ladite région assure un effet de blocage de la partie adjacente de l'élément de renfort. La force de traction exercée sur la seconde zone la plus élastique de ladite région allonge celle-ci davantage que la première zone la moins élastique de sorte que la partie adjacente de l'élément de renfort correspondant suit la déformation de la seconde zone la plus élastique et s'incline vers l'une des extrémités de la ceinture proportionnellement à la force de traction exercée sur lesdites extrémités par le patient et en fonction de sa morphologie.

De préférence, les éléments de renfort longilignes et inclinables sont décalés, de manière symétrique, de l'axe médian de la ceinture, en sorte que lorsque l'usager ajuste la ceinture à sa taille et exerce une traction sur ses extrémités pour faire coopérer les moyens de fermeture, chaque élément de renfort s'incline vers l'extrémité la plus proche selon le même déplacement angulaire.

Des essais menés sur des patients ont mis en avant l'amélioration du confort des dispositifs de soutien selon la présente invention et corrélativement de l'effet thérapeutique escompté puisque les patients portent pendant le temps préconisé lesdits dispositifs.

Lorsque l'usager ajuste la ceinture à sa taille et exerce une traction sur chacune de ses extrémités pour faire coopérer les moyens complémentaires de fermeture, chacun des éléments de renfort latéraux s'incline vers l'extrémité de la ceinture qui lui est le plus proche. Les éléments de renfort latéraux ainsi inclinés adoptent des directions sensiblement parallèles aux lignes virtuelles reliant respectivement les hanches droite et gauche aux cotés droit et gauche de la taille.

La région à élasticité différenciée décrite ci-dessus, comportant une première zone, est disposée entre lesdits éléments de renfort latéraux.

Les éléments de renfort latéraux peuvent être fixés sur la ceinture, par exemple par couture ou soudure diélectrique (type ultrasons ou haute-fréquences). Les éléments de renfort latéraux peuvent être dans une position légèrement inclinée en sorte de former un « V » inversé ou de préférence, sensiblement parallèles et disposés à la verticale du patient lorsque celui-ci est debout. Le plastron dorsal n'est pas directement fixé sur la ceinture ; il l'est indirectement par l'intermédiaire des éléments de renfort latéraux dont il assure la liaison.

Les extrémités inférieures des éléments de renfort n'étant pas bloquées par le plastron dorsal, elles sont aptes à pivoter vers l'une des extrémités de la ceinture en sorte d'adopter une inclinaison adaptée à la morphologie du patient, en particulier à la proportion hanche-taille définissant les lignes virtuelles reliant les hanches à la taille.

De préférence l'élasticité différenciée entre les éléments de renfort latéraux, selon la hauteur de la ceinture, est croissante de son bord supérieur à son bord inférieur.

De préférence, le plastron dorsal n'est solidaire de la ceinture que dans sa partie supérieure reliée aux extrémités supérieures des éléments de renfort latéraux. Plus particulièrement, les bords gauche et droit du plastron dorsal sont solidaires respectivement du bord droit d'un premier élément de renfort latéral et du bord gauche d'un second élément de renfort latéral.

Dans une variante, le plastron dorsal comporte deux éléments complémentaires de renfort longilignes et centraux.

Dans une variante, chaque élément de renfort ou chaque élément complémentaire de renfort longiligne est une baleine insérée dans un fourreau fixé sur la hauteur de la ceinture ou du plastron dorsal.

Dans une variante, le plastron dorsal est relié aux éléments de renfort latéraux sur environ un tiers de leur hauteur totale.

Dans une variante, la ceinture est formée de deux bandes extensibles pourvues à l'une de leurs extrémités des moyens complémentaires de fermeture, et liées par l'intermédiaire des dits éléments de renfort latéraux solidarisées dans leurs extrémités supérieures par la ou les dites pièce(s) de blocage.

Le nombre de renfort latéraux est de préférence pair et est fonction du confort, du coût et du soutien visé.

Dans une variante, la ceinture comporte deux bandes extensibles qui convergent depuis les éléments de renfort latéraux vers les extrémités de ladite ceinture.

L'angle de convergence des bandes est fonction de la pathologie à traiter.

De préférence, pour un pivotement angulaire amélioré et contrôlé, la ceinture comporte une pièce élastique de rappel, éventuellement formée d'une ou plusieurs bandes élastiques, solidarisée et disposée entre les éléments de renfort latéraux. Ladite pièce élastique peux présenter une élasticité homogène sur toute sa hauteur ou croissante de son bord supérieur à son bord inférieur.

Dans ce cas, de préférence, le plastron dorsal est agencé sur la face interne du dispositif et la pièce élastique de rappel est agencée sur la face externe du dispositif en sorte de recouvrir tout ou partie du plastron dorsal lors de son utilisation.

L'invention sera mieux comprise, grâce à la description ci-après, qui se rapporte à deux modes de réalisation préférés, donnés à titre d'exemples non limitatifs, et expliqués avec références aux dessins schématiques annexés, dans lesquels :
- la figure 1 est une vue de la face intérieure d'un premier exemple de dispositif selon la présente invention à plat ;
- la figure 2 est une vue de la face extérieure du dispositif représenté à la figure 1 ;
- la figure 3 est une vue en perspective du dispositif représenté aux figures 1 et 2 porté sur un patient ;
- la figure 4 est une vue de la face intérieure d'un second exemple de dispositif selon la présente invention à plat ;
- la figure 5 est une vue du second exemple de dispositif représenté à la figure 4 porté sur un patient.

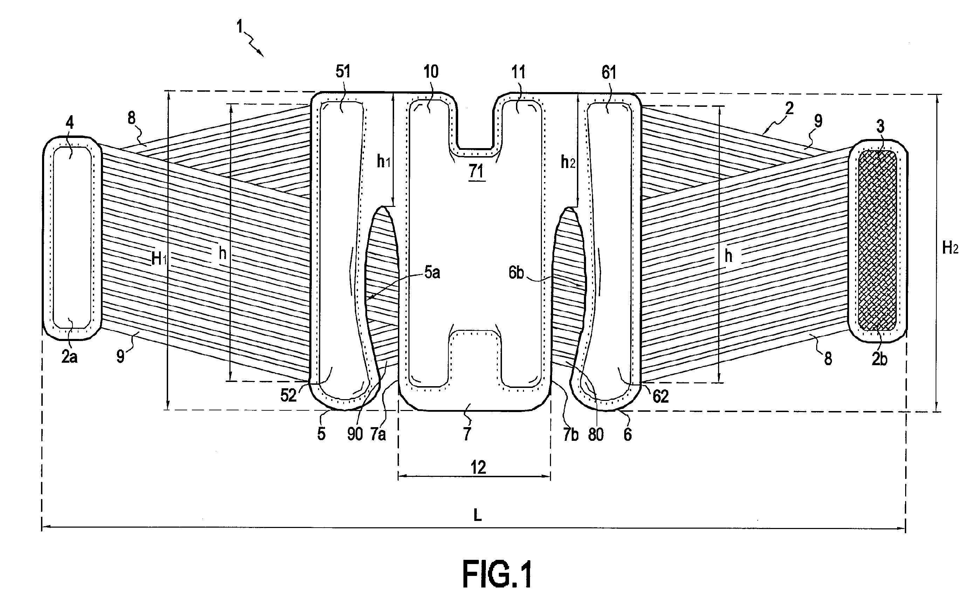

Le dispositif de soutien lombaire 1 représenté à la figure 1 comporte une ceinture élastique 2 munie à ses extrémités 2a,2b de moyens complémentaires de fermeture 3,4, de préférence du type à boucles et à crochets. Le dispositif de soutien 1 comprend des premier 5 et second 6 éléments de renfort latéraux et longilignes disposés de part d'autre d'un plastron dorsal 7. Les éléments de renfort latéraux 5,6 sont fixés sur la hauteur h de la ceinture 2 par n'importe quel moyen : couture, soudure ultra-sons ou haute fréquences. Les éléments de renfort latéraux 5,6 ont respectivement une hauteur déterminée H1 et H2, de préférence du même ordre. La ceinture 2 comporte, de part et d'autre des deux éléments de renfort latéraux, deux bandes extensibles 8,9 qui convergent depuis les deux éléments de renfort latéraux 5,6 vers ses extrémités 2a,2b. La ceinture 2 est ainsi extensible d'une extrémité à l'autre. Le plastron dorsal 7 s'étend sur la hauteur h de la ceinture 2 et comporte deux éléments de renfort complémentaires, longilignes et centraux 10,11. Les bords droit 7a et gauche 7b du plastron dorsal 7 sont respectivement reliés au bord gauche 5a du premier élément de renfort latéral 5 et au bord droit 6b du second élément de renfort latéral 6 et ce dans les extrémités supérieures 51,61 desdits élément de renfort latéraux 5,6. Dans cet exemple précis, le plastron dorsal 7 est relié aux éléments de renfort latéraux 5,6 selon environ un tiers de leur hauteur totale H1,H2 correspondant aux hauteurs h1,h2. Les éléments de renfort respectivement latéraux 5,6 et centraux 10,11 sont des baleines insérées dans des fourreaux fixés sur la hauteur respectivement de la ceinture 2 et du plastron dorsal 7. Le plastron dorsal 7 recouvre une région 12 donnée de la longueur L de la ceinture 2 correspondant à une première zone de blocage 71. Dans cet exemple précis, la première zone 71 et la région 12 sont sensiblement centrées sur la longueur L de la ceinture 2. Le plastron dorsal 7 présente globalement une élasticité, notamment un module élastique E1, inférieur à l'élasticité des bandes extensibles 8,9, notamment inférieur à leurs modules élastiques respectifs E2 et E3. De préférence, le plastron dorsal 7 et les éléments de renfort latéraux 5,6 ne sont pas élastiques. La première zone 71 du plastron dorsal 7 bloque ainsi l'allongement des bandes extensibles 8,9 dans ladite région 12 au niveau des extrémités supérieures 51,61 des éléments de renfort latéraux 5,6, de sorte que ladite première zone 71 du plastron dorsal 7 agit telle une pièce de blocage et permet de différencier l'élasticité longitudinale de la ceinture 2 selon sa hauteur h.

Dans cet exemple précis, les éléments de renfort latéraux 5,6 sont parallèles et disposés à la verticale du patient lorsque ce dernier est debout et que la ceinture n'est pas mise en extension.

De préférence, les fourreaux latéraux dans lesquels sont logés les éléments de renfort latéraux 5,6 sont confectionnés à partir d'un matériau moins élastique que les bandes extensibles 8,9, ce matériau étant éventuellement semblable au matériau dans lequel sont confectionnés les fourreaux centraux. De préférence ledit matériau est un textile, en particulier un tissu tridimensionnel dont la face intérieure, destinée à être en contact avec l'utilisateur, comporte des ouvertures de l'ordre du millimètre pour favoriser l'évacuation de la chaleur et de l'humidité.

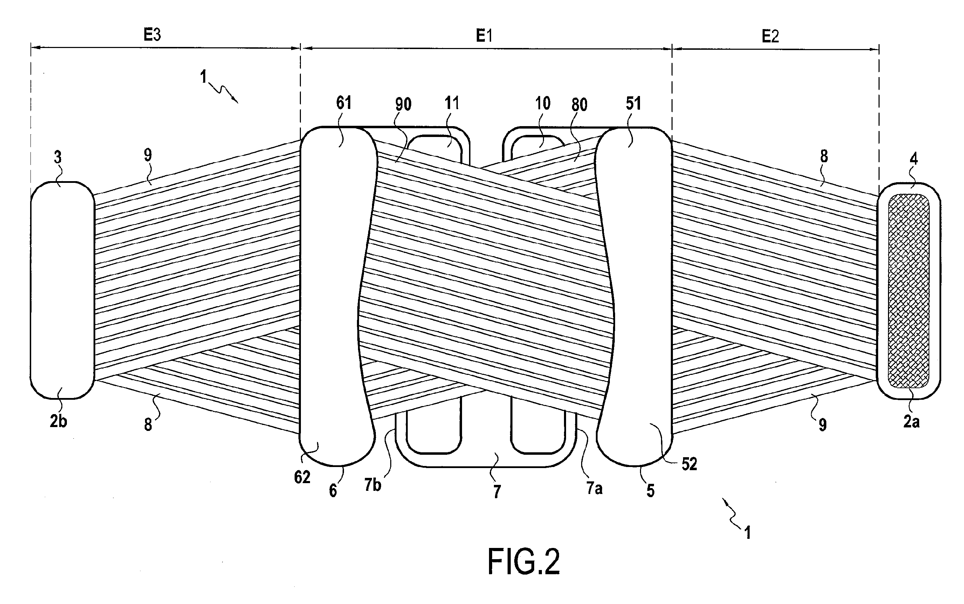

De préférence, comme représenté aux figures 1 à 3, les deux éléments de renfort latéraux 5,6 sont reliés par une pièce élastique de rappel qui, dans cet exemple précis, est constituée par deux bandes extensibles 80,90 qui s'entrecroisent. Cette pièce est destinée à contrôler le pivotement angulaire des deux éléments de renfort latéraux 5,6. Elle peut présenter une élasticité homogène sur toute sa hauteur. Elle peut aussi présenter une élasticité croissante depuis son bord supérieur jusqu'à son bord inférieur, ce qui constitue une solution intermédiaire entre l'absence de pièce de rappel et une pièce de rappel ayant une élasticité homogène sur toute sa hauteur, au regard de la force à exercer pour obtenir un pivotement angulaire donné.

Les éléments de renfort latéraux 5,6 et le plastron dorsal 7 sont agencés sur la face interne du dispositif 1, c'est-à-dire celle qui est tournée vers l'utilisateur lors de son utilisation. La pièce élastique de rappel 80,90 est agencée sur la face externe du dispositif et recouvre tout ou partie du plastron dorsal 7. Ainsi, la pièce de rappel plaque le plastron dorsal sur le dos, améliorant le renfort dorsal sans limiter la liberté de mouvement des extrémités inférieures des éléments de renfort latéraux.

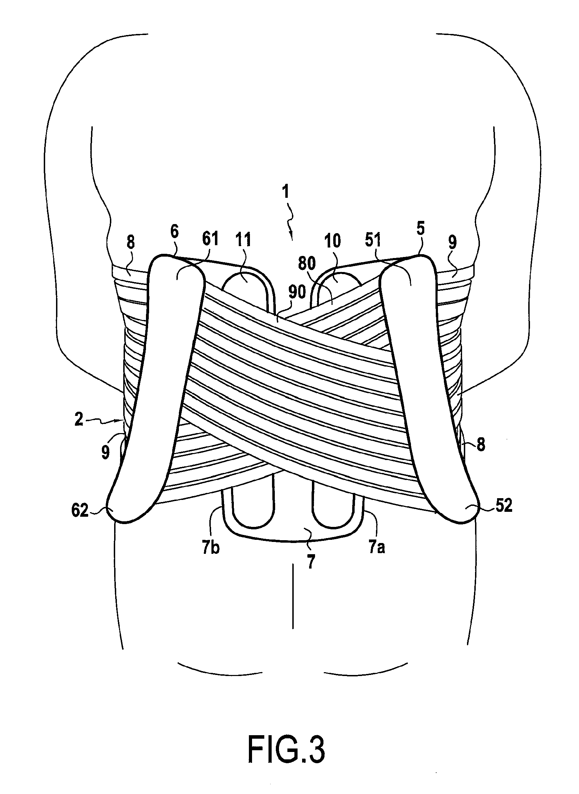

En fonctionnement, l'usager dispose la ceinture 2 sur sa taille et ses hanches puis exerce une traction sur chacune des extrémités 2a,2b de celle-ci. Les forces de traction exercées aux extrémités 2a,2b de la ceinture 2 se propagent aux éléments de renfort latéraux 5,6. Les extrémités supérieures 51,61 des éléments de renfort latéraux 5,6 étant bloquées par la première zone 71 du plastron dorsal 7, faisant office de pièce de blocage, les extrémités inférieures 52,62 des éléments de renfort latéraux 5,6 pivotent angulairement selon leur axe autour de leurs extrémités supérieures 51,61 vers l'extrémité 2a,2b de la ceinture 2 la plus proche. Le patient ferme la ceinture 2 en faisant coopérer les moyens de fermeture 3,4. Les premier 5 et second 6 éléments de renfort latéraux ont alors respectivement des directions sensiblement parallèles aux lignes virtuelles reliant les hanches gauche et droite aux côtés gauche et droit de la taille tels que représentés à la figure 3. Cette disposition des éléments de renfort latéraux 5,6 correspond à un « V » inversé. Les éléments de renfort latéraux 5,6 se sont orientés en fonction de la morphologie du patient, notamment en fonction du rapport entre les mensurations des hanches et de la taille, améliorant ainsi très significativement son confort.

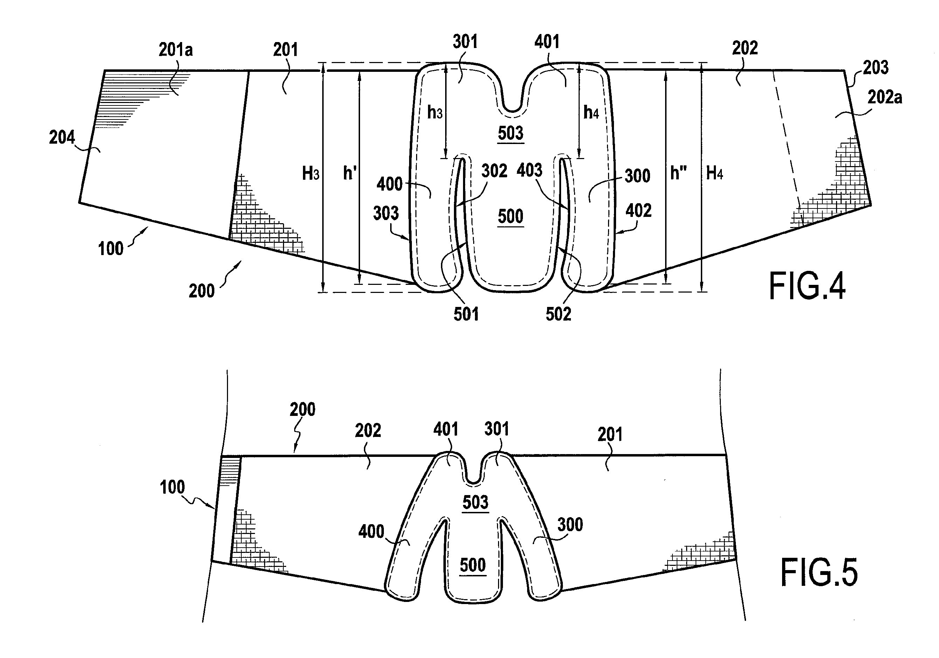

Le second exemple simplifié de dispositif de soutien lombaire 100 représenté aux figures 4 et 5 diffère du dispositif 1 en ce qu'il comprend une ceinture 200 ayant, de part et d'autre des deux éléments de renfort latéraux 300,400, une seule bande extensible 201,202 comportant à son extrémité 201a,202a des moyens complémentaires de fermeture 203,204 et ne comporte pas de pièce élastique de rappel. Les bandes 201,202 sont reliées, par l'intermédiaire de deux éléments de renfort latéraux et longilignes 300,400 solidarisées dans leurs extrémités supérieures 301,401 à un plastron dorsal 500 faisant office de pièce de blocage. Le plastron dorsal 500 peut être formé d'un seul élément de renfort longiligne ou comprendre deux éléments de renfort complémentaires et longilignes.

La hauteur H3,H4 des éléments de renfort latéraux 300,400 est de l'ordre de celles h',h" des bandes extensibles 201,202.

Tel que pour le dispositif 1, les bords droit 501 et gauche 502 du plastron dorsal 500 sont respectivement reliés au bord gauche 302 du premier élément de renfort latéral 300 et au bord droit 403 du second élément de renfort latéral 400 et ce dans les extrémités supérieures 301,401 desdits élément de renfort latéraux 300,400. Dans cet exemple précis, le plastron dorsal 500 est relié aux éléments de renfort latéraux 300,400 selon environ un tiers de leur hauteur totale H3,H4 correspondant aux hauteurs h3,h4.

En fonctionnement, lorsque le patient ajuste la ceinture 200 à sa taille, les éléments de renfort latéraux 300,400 étant, dans cet exemple précis représenté en figure 5, libres selon les deux tiers de leurs hauteurs H3, H4, respectivement selon leurs bords gauche 302 et droit 401, pivotent angulairement autour de leurs extrémités supérieures 301,401 et de la première zone de blocage 503 en sorte d'adopter des lignes virtuelles reliant les hanches aux cotés de la taille respectant la morphologie unique du patient.

## Revendications

1. Dispositif de soutien (1,100), comportant une ceinture élastique (2,200), munie à ses extrémités (2a,2b,201a,202a) de moyens complémentaires de fermeture (3,4,203,204), et au moins deux éléments de renfort latéraux et longilignes (5,6,300,400) fixés sur la hauteur de la ceinture (2,200), entre lesquels l'élasticité de la ceinture est différenciée selon sa hauteur, **caractérisé en ce qu'**il comporte un plastron dorsal (7,500) qui s'étend sur la hauteur de la ceinture, et qui n'est solidaire de la ceinture que dans sa partie supérieure reliée aux extrémités supérieures desdits éléments de renfort latéraux en sorte que la partie supérieure dudit plastron dorsal soit reliée uniquement aux extrémités supérieures desdits éléments de renfort latéraux et fasse office de pièce de blocage apte à limiter l'extensibilité de la ceinture, la mise en extension longitudinale de la ceinture (2,200) provoquant le déplacement angulaire des extrémités inférieures desdits éléments de renfort latéraux (5,6,300,400) vers l'une desdites extrémités (2a,2b, 201a,202a) de la ceinture (2,200).

2. Dispositif (1,100) selon la revendication 1, **caractérisé en ce que** l'élasticité différenciée entre les éléments de renfort latéraux, selon la hauteur de la ceinture, est croissante de son bord supérieur à son bord inférieur.

3. Dispositif (1,100) selon l'une ou l'autre des revendications 1 et 2, **caractérisé en ce que** les éléments de renforts latéraux ne sont solidaires que dans leurs extrémités supérieures par le biais du plastron dorsal.

4. Dispositif (1) selon l'une ou l'autre des revendications 1 et 2, **caractérisé en ce qu'**il comporte une pièce élastique de rappel, éventuellement formée d'une ou plusieurs bandes élastiques (80,90), solidarisée et disposée entre les éléments de renfort latéraux (5,6).

5. Dispositif (1) selon la revendication 4, **caractérisé en ce que** le plastron dorsal (7) est agencé sur la face interne du dispositif et la pièce élastique de rappel (80,90) est agencée sur la face externe du dispositif, en sorte de recouvrir tout ou partie du plastron dorsal (7) lors de l'utilisation du dispositif.

6. Dispositif (1) selon l'une des revendications 1 à 5, **caractérisé en ce que** le plastron dorsal (7) comporte deux éléments complémentaires de renfort (10,11), longilignes et fixés sur la hauteur du plastron (7).

7. Dispositif (1,100) selon l'une des revendications 1 à 6, **caractérisé en ce que** chaque élément de renfort (5,6,300,400) et/ou élément complémentaire (10,11) de renfort est une baleine insérée dans un fourreau fixé sur la hauteur de la ceinture (2,200) ou du plastron dorsal (7,500).

8. Dispositif (1,100) selon l'une des revendications 1 à 7, **caractérisé en ce que** le plastron dorsal (7,500) est relié aux éléments de renfort latéraux (5,6,300,400) sur environ un tiers (h1,h2,h3,h4) de leur hauteur totale (H1,H2,H3,H4).

9. Dispositif (1) selon l'une des revendications 1 à 8, **caractérisé en ce que** la ceinture (2) est formée de deux bandes extensibles (8,9) pourvues à l'une de leurs extrémités (2a,2b) des moyens complémentaires de fermeture (3,4), et sont liées par leur autre extrémité, aux deux éléments de renfort latéraux (5,6).

10. Dispositif (1) selon la revendication 9, **caractérisé en ce que** la ceinture (2) est formée de deux bandes extensibles (8,9) qui convergent vers les extrémités (2a,2b) de ladite ceinture (2).

## Patentansprüche

1. Stützvorrichtung (1, 100), umfassend einen elastischen Gürtel (2, 200), der an seinen Enden (2a, 2b, 201 a, 202a) mit ergänzenden Schließmitteln (3, 4, 203, 204) versehen ist, sowie wenigstens zwei seitliche und langgestreckte, über die Höhe des Gürtels (2, 200) befestigte Verstärkungselemente (5, 6, 300, 400), zwischen denen die Elastizität des Gürtels je nach seiner Höhe differenziert ist, **dadurch gekennzeichnet, dass** sie ein Rückenelement (7, 500) umfasst, das sich über die Höhe des Gürtels erstreckt und das mit dem Gürtel nur in seinem oberen Teil, der mit den oberen Enden der seitlichen Verstärkungselemente verbunden ist, fest verbunden ist, so dass der obere Teil des Rückenelements lediglich mit den oberen Enden der seitlichen Verstärkungselemente verbunden ist und als Blockierteil dient, das geeignet ist, die Dehnbarkeit des Gürtels zu begrenzen, wobei das Längsdehnen des Gürtels (2, 200) die Winkelbewegung der unteren Enden der seitlichen Verstärkungselemente (5, 6, 300, 400) in Richtung von einem der Enden (2a, 2b, 201 a, 202a) des Gürtels (2, 200) bewirkt.

2. Vorrichtung (1, 100) nach Anspruch 1, **dadurch gekennzeichnet, dass** die differenzierte Elastizität zwischen den seitlichen Verstärkungselementen, je nach Höhe des Gürtels, von seinem oberen Rand zu seinem unteren Rand zunehmend ist.

3. Vorrichtung (1, 100) nach dem einen oder anderen der Ansprüche 1 und 2, **dadurch gekennzeichnet, dass** die seitlichen Verstärkungselemente nur in ihren oberen Enden mittels des Rückenelements fest verbunden sind.

4. Vorrichtung (1) nach dem einen oder anderen der Ansprüche 1 und 2, **dadurch gekennzeichnet, dass** sie ein eventuell von einem oder mehreren elastischen Bändern (80, 90) gebildetes elastisches Rückstellteil umfasst, das zwischen den seitlichen Verstärkungselementen (5, 6) fest verbunden und angeordnet ist.

5. Vorrichtung (1) nach Anspruch 4, **dadurch gekennzeichnet, dass** das Rückenelement (7) auf der Innenseite der Vorrichtung angeordnet ist und das elastische Rückstellteil (80, 90) auf der Außenseite der Vorrichtung angeordnet ist, so dass es bei der Verwendung der Vorrichtung das gesamte Rückenelement (7) oder einen Teil dessen bedeckt.

6. Vorrichtung (1) nach einem der Ansprüche 1 bis 5, **dadurch gekennzeichnet, dass** das Rückenelement (7) zwei ergänzende, langgestreckte und über die Höhe des Elements (7) befestigte Verstärkungselemente (10, 11) umfasst.

7. Vorrichtung (1, 100) nach einem der Ansprüche 1 bis 6, **dadurch gekennzeichnet, dass** jedes Verstärkungselement (5, 6, 300, 400) und/oder ergänzende Verstärkungselement (10, 11) ein Stab ist, der in eine über die Höhe des Gürtels (2, 200) oder des Rückenelements (7, 500) befestigte Hülse eingefügt ist.

8. Vorrichtung (1, 100) nach einem der Ansprüche 1 bis 7, **dadurch gekennzeichnet, dass** das Rückenelement (7, 500) mit den seitlichen Verstärkungselementen (5, 6, 300, 400) über etwa ein Drittel (h1, h2, h3, h4) ihrer Gesamthöhe (H1, H2, H3, H4) verbunden ist.

9. Vorrichtung (1) nach einem der Ansprüche 1 bis 8, **dadurch gekennzeichnet, dass** der Gürtel (2) von zwei dehnbaren Bändern (8, 9) gebildet ist, die an einem ihrer Enden (2a, 2b) mit den ergänzenden Schließmitteln (3, 4) versehen sind und mit ihrem anderen Ende mit den beiden seitlichen Verstärkungselementen (5, 6) verbunden sind.

10. Vorrichtung (1) nach Anspruch 9, **dadurch gekennzeichnet, dass** der Gürtel (2) von zwei dehnbaren Bändern (8, 9), die in Richtung der Enden (2a, 2b) des Gürtels (2) zusammenlaufen, gebildet ist.

## Claims

1. A support device (1, 100), which device comprises an elastic belt (2, 200) provided, at its ends (2a, 2b, 201a, 202a), with complementary closure means (3, 4, 203, 204), and at least two elongate side reinforcing elements (5, 6, 300, 400) that are fastened over the height of the belt (2, 200), and between which side reinforcing elements the longitudinal elasticity of the belt varying over the height thereof, said support device being **characterized in that** it further comprises a back panel that extends over the height of the belt and that is secured to the belt in its top portion only that is secured to the top ends of said side reinforcing elements in order that said top portion of the back panel is connected to the top ends only of said side reinforcing elements and so as to act as a restraining piece suitable for limiting the extensibility of the belt so that extending the belt (2, 200) longitudinally causes the bottom ends of said side reinforcing elements (5, 6, 300, 400) to shift angularly towards respective ones of said ends (2a, 2b, 201a, 202b) of the belt (2, 200).

2. A device (1,100) according to claim 1, **characterized in that**, between the side reinforcing elements, the elasticity of the belt that varies over the height thereof increases going from the top edge to the bottom edge thereof.

3. A device (1, 100) according to claim 1 or claim 2, **characterized in that** the side reinforcing elements are secured together at their top ends only, via the back panel.

4. A device (1) according to claim 1 or claim 2, **characterized in that** it further comprises an elastic return piece that is optionally made up of one or more elastic strips (80, 90), and that is disposed between the side reinforcing elements (5, 6).

5. A device (1) according to claim 4, **characterized in that** the back panel (7) is arranged on the inside face of the device, and the elastic return piece (80, 90) is arranged on the outside face of the device, so as to cover all or some portion of the back panel (7) while the device is being used.

6. A device (1) according to any one of claims 1 to 5, **characterized in that** the back panel (7) is made up of two complementary elongate reinforcing elements (10, 11) that are fastened over the height of the back panel (7).

7. A device (1, 100) according to any one of claims 1 to 6, **characterized in that** each reinforcing element (5, 6, 300, 400) and/or each complementary elongate reinforcing element (10, 11) is a stay inserted into a sheath fastened over the height of the belt (2, 200) or over the height of the back panel (7, 500).

8. A device (1, 100) according to any one of claims 1 to 7, **characterized in that** the back panel (7, 500) is connected to the side reinforcing elements (5, 6, 300, 400) over about one third (hi, h2, h3, h4) of the total height (H1, H2, H3, H4) thereof.

9. A device (1) according to any one of claims 1 to 8, **characterized in that** the belt (2) is made up of two extensible strips (8, 9) that, at respective ones of their ends (2a, 2b), are provided with the complementary closure means (3, 4), and, at their respective other ends, are secured to respective ones of the two side reinforcing elements (5, 6).

10. A device (1) according to claim 9, **characterized in that** the belt (2) is made up of two extensible strips (8, 9) that converge towards the ends (2a, 2b) of said belt (2).
